# DEMANDE DE BREVET EUROPEEN

(11) **EP 0 864 359 A1**
(43) Date de publication de la demande: **16.09.1998**
(21) Numéro de dépôt: 98400518.1
(22) Date de dépôt: 04.03.1998
(51) Int. Cl.: B01J 23/648, B01J 23/62, C07C 5/333

(54) **Procédé et composition catalytique pour la déshydrogénation d'alcanes en alcènes**

(30) Priorité: 13.03.1997 FR 9702998
(71) Demandeur: TOTAL RAFFINAGE DISTRIBUTION S.A., 92800 Puteaux (FR)
(72) Inventeur: Szabo, Georges, 76290 Montivilliers (FR); Meriaudeau, Paul, 01160 Pont D'Ain (FR); Sapaly, Gilbert, 69009 Lyon (FR)
(74) Mandataire: Jolly, Jean-Pierre

(57) **Abrégé**

L'invention a pour objet procédé amélioré pour la déshydrogénation des alcanes en alcènes.

Ce procédé consiste à faire passer la charge dans au moins un réacteur en présence d'au moins un catalyseur de déshydrogénation. et se caractérise en ce que l'on emploie un catalyseur comprenant un support d'oxyde réfractaire inorganique, au moins un métal du groupe du platine ainsi que du niobium sous forme d'oxyde et à une teneur comprise entre 5% et 70% en poids de niobium par rapport au poids dudit catalyseur

L'invention concerne également une composition catalytique améliorée ainsi qu'un procédé de préparation et d'activation de catalyseurs pouvant être utilisés dans le procédé de déshydrogénation des alcanes en alcènes.

## Description

La présente invention a pour objet un procédé amélioré pour la déshydrogénation des alcanes en alcènes. L'invention concerne en particulier l'utilisation de compositions catalytiques originales pour la déshydrogénation d'alcanes comportant de 2 à 15 atomes de carbone en oléfines correspondantes et en hydrogène.

Le procédé de déshydrogénation des alcanes en alcènes est utilisé industriellement. Ce procédé consiste faire réagir des alcanes en présence d'une composition catalytique de déshydrogénation. Deux familles de compositions catalytiques sont employées: la première contenant du chrome supporté sur alumine et la seconde contenant du platine supporté sur alumine. On sait également que la conversion en alcènes est d'autant plus difficile que la chaîne carbonée de l'alcane utilisé est plus courte.

De nombreuses compositions catalytiques de déshydrogénation sont décrites dans la littérature. Généralement, les compositions catalytiques contiennent des métaux tels que le platine, le chrome, le gallium, etc... Les métaux sont incorporés à des matériaux formant le support ou la matrice du catalyseur ; les matériaux les plus couramment employés sont les alumines, les aluminosilicates, les silices ou les silicalites.

La conversion des alcanes en alcènes tels que l'éthylène, le propylène, le butylène et l'isobutylène, est essentielle, notamment dans le domaine de l'industrie du pétrole pour la valorisation de certaines coupes pétrolières. En particulier, l'isobutène et l'isopentène permettent la préparation du méthyl-tertio-butyl-éther et du tertio-amyl-méthyl-éther, bases utilisées pour augmenter l'indice d'octane des essences, et les oléfines à longue chaîne sont requises pour la préparation la formation des alkylbenzènes linéaires utilisés comme base de détergents.

Toutefois, les compositions catalytiques habituellement mises en oeuvre ont une durée de vie relativement courte, ce qui oblige à les régénérer ou à les remplacer très souvent si l'on veut maintenir la conversion à des rendements élevés.

A titre d'exemple, les documents cités ci-dessous illustrent les multiples tentatives qui ont été faites pour améliorer les performances des compositions catalytiques intervenant dans les procédés de déshydrogénation d'alcanes.

Le brevet US 5 147 837 présente une composition catalytique non acide de déshydrogénation comprenant un métal du groupe du platine, un matériau cristallin microporeux dont la structure est de préférence celle d'une zéolite ZSM-5, du titane et en option de l'étain, de l'indium, du plomb ou du thallium.

Le document FR 2 115 860 décrit un catalyseur contenant 0,01 à 5 % en poids d'un métal du groupe du platine fixé sur un support réfractaire et contenant également environ 0,01 à 5 % en poids de tantale ou de niobium. Le composé du niobium peut être incorporé sous forme d'oxyde solide ou sous forme d'une solution de sel.

Le document EP 212 850 présente une composition catalytique de déshydrogénation comprenant un métal du groupe du platine, de la silicalite comme support, mais sans métal alcalin ou alcalino-terreux.

Le document EP 351 066 présente une composition catalytique de déshydrogénation comprenant également un support de silicalite sur lequel est déposé un métal du groupe du platine. La sélectivité de ce catalyseur est augmentée par l'incorporation d'étain (de 0,1 à 15% en poids). On observe en effet une diminution significative des réactions secondaires, telles que les réactions de craquage et d'oligomérisation, nuisibles car responsables de la formation de sous produits indésirables.

Cependant, si bonnes soient-elles à l'origine, les performances de ces catalyseurs de l'art antérieur se dégradent rapidement: ces compositions catalytiques se désactivent rapidement au cours de leur utilisation, surtout en l'absence d'hydrogène, et présentent donc une stabilité insuffisante. Les procédés mettant en oeuvre ces compositions catalytiques se révèlent donc peu viables économiquement, puiqu'il faut régulièrement renouveler le catalyseur.

Poursuivant ses recherches dans le domaine des catalyseurs de déshydrogénation, la Demanderesse a constaté que, de manière surprenante, l'incorporation d'oxyde de niobium en quantité élevée permettait d'accroitre considérablement la durée de vie des catalyseurs de déshydrogénation.

Pour d'autres familles de catalyseurs, tels que les catalyseurs de type acido-basiques, l'incorporation d'oxyde de niobium est connue en soi.

Ainsi la demande de brevet EP 271 182 décrit un catalyseur de conversion de l'acétone en MIBK (méthyl-isobutyl-cétone). Ce catalyseur, comprenant du palladium déposé sur un support d'alumine et d'oxyde de niobium, permet d'augmenter notablement les rendements de conversion de l'acétone par rapport aux catalyseurs jusqu'alors employés dans ce domaine. Il présente une excellente activité dans les conditions de la réaction de condensation de l'acétone, à savoir à basse température (entre 80 et 250°C) et en présence d'hydrogène. D'après ce document, l'incorporation d'oxyde de niobium permet ainsi d'augmenter la réactivité du catalyseur de conversion de l'acétone, qui est un catalyseur de type acido-basique et hydrogénant, actif à basse température.

Cependant, la Demanderesse a découvert que, de manière totalement inattendue, l'oxyde de niobium a également pour propriété d'augmenter la durée de vie des catalyseurs de déshydrogénation, actifs à haute température.

Ainsi, pour les réactions de déshydrogénation d'alcanes, la Demanderesse préconise l'emploi d'un catalyseur supporté contenant, en plus des ingrédients classiques des catalyseurs de déshydrogénation (oxydes réfractaires inorganiques et métaux du groupe du platine), une quantité élevée d'oxyde de niobium.

La présente invention a par conséquent pour objet un procédé de traitement d'une charge hydrocarbonée contenant une quantité substantielle d'alcanes, par déshydrogénation d'au moins une fraction desdits alcanes en alcènes et hydrogène, procédé dans lequel on fait passer la charge dans au moins un réacteur en présence d'au moins un catalyseur de déshydrogénation. Ce procédé se caractérise en ce que l'on emploie un catalyseur comprenant un support d'oxyde réfractaire inorganique, au moins un métal du groupe du platine ainsi que du niobium sous forme d'oxyde et à une teneur comprise entre 5% et 70% en poids de niobium par rapport au poids dudit catalyseur.

Le procédé selon l'invention permet de déshydrogéner des alcanes en alcènes avec une excellente sélectivité, tout en assurant des rendements stables au cours du temps. En effet, le catalyseur employé fait preuve d'une activité remarquablement stable au cours de la réaction, et assure des vitesses réactionnelles élevées.

Ainsi, grâce à l'utilisation la composition catalytique selon l'invention, le procédé de déshydrogénation ne nécessite pas un apport d'hydrogène exogène ou d'un autre gaz diluant visant à maintenir l'efficacité du catalyseur, comme c'était souvent le cas dans les procédés de l'art antérieur.

La présente invention concerne également des compositions catalytiques susceptibles d'être employées dans le procédé mentionné ci-avant, et qui s'avèrent particulièrement performantes pour la déshydrogénation des alcanes en alcènes et hydrogène.

La présente invention a enfin pour objet des méthodes de préparation et d'activation des catalyseurs intervenant dans le procédé selon l'invention, méthodes permettant d'améliorer sensiblement les performances desdits catalyseurs dans les conditions spécifiques de la réaction de déshydrogénation des alcanes.

Dans le procédé selon l'invention, on emploie un catalyseur contenant un support comprenant au moins un oxyde réfractaire inorganique d'un élément du groupe II, III, ou IV de la classification périodique des éléments, un mélange de ces oxydes ou un composé contenant un ou plusieurs de ces oxydes. Les oxydes individuels peuvent être une silice, une alumine, une silice-alumine, une silicalite, un alumino-silicate, une magnésie, une zircone, une silice-oxyde de magnésium ou une alumine-oxyde de bore, l'oxyde préféré étant la silicalite.

Le catalyseur comprend également au moins un métal du groupe du platine, à savoir le groupe constitué par le rhuténium, le rhodium, le palladium, l'osmium, l'iridium et le platine.

De préférence, on utilise le platine. La source de platine est de préférence choisie parmi l'acide chloroplatinique de formule PtH₂Cl₆ et les sels de platine solubles tels que l'hydroxyde de tétramine de platine de formule Pt(NH₃)₄(OH)₂, et le chlorure de tétramine de platine de formule Pt(NH₃)₄Cl₂.

La teneur en platine, exprimée en pourcentage massique par rapport à la masse totale de la composition catalytique, est de préférence comprise entre 0,01 et 5 % et plus particulièrement entre 0,01 et 2%.

La teneur en niobium du catalyseur est de préférence inférieure à 50 % en poids. Ainsi, le rapport massique Nb/Pt, c'est-à-dire le rapport des teneurs massiques en niobium et en platine est généralement compris entre 1 et 7000 et de préférence entre 2,5 et 5000.

La source de niobium peut être de l'oxyde de niobium, du chlorure de niobium ou de l'éthoxyde de niobium.

Le procédé de conversion des alcanes en alcènes selon l'invention est mis en oeuvre à des températures comprises entre 300 et 800°C, à une pression comprise entre 10³ Pa et 10⁶ Pa et à un débit massique de la charge par rapport à la masse de catalyseur (pph) de 1 à 50 h⁻¹. De préférence, les températures sont comprises entre 500°C et 600°C et la pression est de l'ordre de 10⁵ Pa.

Puisque la réaction de déshydrogénation est endothermique et est limitée par l'équilibre thermodynamique, le procédé est habituellement conduit à haute température et basse pression pour obtenir des taux élevés de conversion. Sous ces conditions sévères, il est habituellement difficile de maintenir une haute activité et une bonne sélectivité sur de longues périodes, parce que les réactions parasites telles que l'aromatisation, le craquage, l'isomérisation et la formation de coke se développent. Toutefois, si les conditions précitées de température et de pression sont respectées, l'activité et la stabilité des nouvelles compositions catalytiques sont améliorées et la sélectivité de production d'alcènes à partir d'alcanes est optimisée.

Lors de la mise en oeuvre du procédé de conversion, on peut également utiliser un gaz diluant tel que par exemple l'hydrogène, la vapeur d'eau, un gaz inerte (par exemple l'azote), le méthane, etc., pour diluer la charge d'alcanes. Toutefois, l'emploi des compositions catalytiques précitées fait que le recours à un gaz diluant n'est plus obligatoire et la conversion s'effectue donc de préférence en l'absence de diluant.

La conversion en présence des compositions catalytiques de l'invention peut s'effectuer sans dommage en l'absence d'H₂ exogène. Toutefois, si l'on souhaite améliorer la stabilité du catalyseur, la réaction peut être réalisée en présence d'H₂ exogène dans le milieu réactionnel.

Une composition catalytique particulièrement avantageuse pour la déshydrogénation des alcanes en alcènes et hydrogène contient un support de silicalite, au moins un métal du groupe du platine à une teneur comprise entre 0,01 et 5 % en poids, et se caractérise en ce qu'elle contient également du niobium sous forme d'oxyde et à une teneur comprise entre 5% et 70% en poids de niobium par rapport au poids du catalyseur.

Le catalyseur peut avantageusement contenir soit du germanium, soit de l'étain, soit de l'indium, soit un mélange de deux au moins de ces métaux.

La teneur en germanium est de préférence comprise entre 0,01 et 10% en poids et plus particulièrement entre 0,01 et 2 % en poids.

La source de germanium peut être choisie parmi les sels de germanium solubles dans l'eau tels que l'éthoxyde de germanium ou le chlorure de germanium.

De préférence, la source de germanium est le tétraéthoxyde de germanium de formule Ge(OC₂H₅)₄.

La teneur en étain est de préférence comprise entre 0,01 et 10 % en poids et plus particulièrement entre 0,01 et 2 % en poids.

La teneur en indium est de préférence comprise entre 0,01 et 10 % en poids et plus particulièrement entre 0,01 et 2 % en poids.

La source d'indium peut être choisie parmi les sels d'indium solubles dans l'eau tels que le nitrate d'indium, le chlorure d'indium et le sulfate d'indium. De préférence, la source d'indium est le nitrate d'indium de formule In(NO₃)₃.

En présence de la composition catalytique selon l'invention, la conversion et la sélectivité de la réaction de déshydrogénation des alcanes en alcènes sont très élevées. Notamment, dans le cas de la préparation d'isobutène à partir d'isobutane, la conversion et la sélectivité sont respectivement de l'ordre de 40 % et de 98 %, conduisant à un rendement global de l'ordre de 39 %.

En outre, la composition catalytique selon l'invention présente l'avantage de rester très performante après plusieurs cycles d'utilisation et de régénération.

Les catalyseurs susceptibles d'être employés dans le prodédé de déshydrogénation selon l'invention peuvent être préparés selon différentes méthodes connues. Toutefois, un procédé perfectionné de préparation et d'activation a été développé, permettant d'obtenir des compositions catalytiques stables au cours de la réaction de conversion des alcanes en alcènes, tout en augmentant la sélectivité de cette réaction.

Ce procédé de préparation et d'activation comprend préférentiellement les étapes suivantes:
(1) formation d'un support catalytique contenant éventuellement du germanium, de l'indium et/ou de l'étain, en présence d'un agent structurant;
(2) après cristallisation du mélange homogène à pression autogène et à une température comprise entre 25 et 190°C, filtration et lavage à l'eau du matériau solide, puis séchage;
(3) calcination du matériau séché, sous atmosphère inerte, puis en présence d'oxygène ou d'air à une température comprise entre 400 et 700°C;
(4) imprégnation du matériau calciné refroidi avec une solution d'au moins une source d'un métal du groupe du platine, sous agitation constante, à une température comprise entre 15 et 95°C ;
(5) évaporation de la phase liquide;
(6) activation du solide récupéré par (i) traitement sous oxygène ou air à une température croissante de 15 à 320°C et (ii) traitement sous hydrogène à une température croissante de 320 à 550°C; et,
(7) refroidissement du catalyseur obtenu;
(8) mélange mécanique du catalyseur obtenu avec de l'oxyde de niobium.

Une variante du procédé de préparation et d'activation comprend les étapes suivantes :
(1) formation d'un support catalytique contenant du niobium et éventuellement du germanium, de l'indium et/ou de l'étain, en présence d'un agent structurant;
(2) après cristallisation du mélange homogène à pression autogène et à une température comprise entre 150 et 190°C, filtration et lavage à l'eau du matériau solide, puis séchage;
(3) calcination du matériau séché, sous atmosphère inerte, puis en présence d'oxygène ou d'air à une température comprise entre 400 et 700°C;
(4) imprégnation du matériau calciné refroidi avec une solution d'au moins une source d'un métal du groupe du platine, sous agitation constante, à une température comprise entre 25 et 90°C ;
(5) récupération du solide par évaporation de la phase liquide ou par centrifugation;
(6) activation du solide récupéré par (i) traitement sous oxygène ou air à une température croissante de 20 à 320°C et (ii) traitement sous hydrogène à une température croissante de 320 à 550°C; et,
(7) refroidissement du catalyseur obtenu;
(8) mélange mécanique du solide ainsi obtenu avec de l'oxyde de niobium.

La cristallisation du mélange dépend d'un certain nombre de facteurs, notamment de la température. Par exemple, si la température est de l'ordre de 170°C, on laisse de préférence la cristallisation s'opérer pendant 1 à 4 jours.

L'étape d'imprégnation (4) s'effectue avantageusement pendant 12 heures; un acide minéral peut être ajouté à la solution, par exemple l'acide nitrique, pour faciliter la dispersion du métal du groupe du platine. De préférence, l'accroissement de la température dans l'étape d'activation (6) est de 0,2°C par minute pour l'oxydation puis de 1°C par minute pour la réduction.

L'agent structurant peut être choisi parmi les amines telles que la diéthylamine, le diamino-1,6-hexane, les alcanolamines telles que la diéthanolamine, les sels d'ammonium tels que le bromure de tétrapropylammonium (TPABr) ou l'hydroxyde de tétrapropylammonium (TPAOH), et les composés similaires.

L'invention est illustrée ci-après par des exemples de préparation de compositions catalytiques ainsi que par des tests comparatifs de procédés mettant en oeuvre des catalyseurs de l'art antérieur et des catalyseurs préconisés dans l'invention.

### EXEMPLES

Les exemples 1, 2, 3 et 4 illustrent la préparation de compositions catalytiques selon l'art antérieur et les exemples 5, 6, 7 et 8 illustrent des compositions catalytiques selon l'invention.

La structure observée aux rayons X du support de chaque catalyseur préparé est celle de la silicalite 1 précisée dans la description.

### Exemple 1

Une composition catalytique A supportée sur de la silicalite et contenant du platine et du niobium sous forme de Nb₂O₅, a été préparée selon le mode de préparation suivant :
30 g de silicate de sodium sont mis en solution dans 50 ml d'eau à laquelle on ajoute 3 g de tétrapropylammonium bromure (TPABr). A cette solution, on ajoute sous agitation jusqu'à complète homogénéisation 30 ml d'eau contenant 1,9 g de SO₄H₂ (solution à 1N) et 0,2 g d'éthoxyde de niobium Nb(OC₂H₅)₅. Le mélange homogène est transféré dans un autoclave, chauffé à 180 °C sous pression autogène pendant 3 jours.

Après cristallisation, le solide est lavé puis calciné sous azote à une température augmentant de 0,2 °C/min et allant de 25 °C à 550 °C puis le solide est placé sous O₂ et laissé à cette température pendant 12 heures. Le solide ainsi préparé présente aux rayons X la structure de la silicalite.

10 g de ce solide sont ensuite ajoutés dans une solution de 100 ml d'eau contenant 76 mg de Pt(NH₃)₄(OH)₂. Après 24 heures d'agitation, l'eau est évaporée et le solide est séché à 80 °C.

L'activation du catalyseur s'effectue sous courant de O₂, la température augmentant de 0,2 °C/min jusqu'à 320 °C et un palier de 8 heures est observé à 320 °C. Ensuite, après avoir effectué un balayage du solide à l'azote puis à l'hydrogène, la température est augmentée de 1 °C/min jusqu'à 550 °C.

La composition catalytique A ainsi préparée contient, sur silicalite, 0,5% en poids de platine et 1 % en poids de niobium.

### Exemple 2

Une composition catalytique B supportée sur silicalite et contenant du platine, du germanium et de l'indium, a été préparée selon le mode de préparation suivant :
a) 20 g de silicate de sodium sont mis en solution dans 50 g d'eau à laquelle on ajoute ensuite 3 g de TPABr (bromure de tétrapropylammonium),
b) on ajoute à cette solution maintenue sous agitation jusqu'à homogénéisation 30 g d'eau contenant 1,9 g d'H₂SO₄ (solution 1N), 0,3 g de Ge(OC₂H₅)₄ (tétraéthoxyde de germanium) et 0,2 g de nitrate d'indium.
c) le mélange homogénéisé est transféré dans un autoclave qui est ensuite chauffé à 175 °C sous pression autogène pendant 3 jours,
d) après lavage et séchage, le solide est calciné sous azote à une température augmentant de 0,2°C par min et allant de 25°C à 550°C, puis placé sous O₂ et laissé à cette température de 550°C pendant 12 heures; le solide obtenu a la structure de la silicalite 1, comme observé aux rayons X,
e) 10 g du solide sont ensuite ajoutés dans une solution de 100 g d'eau contenant 76 mg de Pt(NH₃)₄(OH)₂,
f) après 24 heures d'agitation à 75°C, l'eau est évaporée et le solide est séché à 80°C,
g) l'activation du catalyseur obtenu s'effectue sous courant d'O₂ à une température progressant de 0,2°C par min et allant de 25 à 320°C, puis pendant 8 heures à cette température,
h) après balayage à l'azote, le catalyseur est réduit sous H₂ à une température progressant de 1°C par minute et allant de 320 à 550°C.

La composition catalytique B ainsi obtenue contient, sur silicalite, 0,5% en poids de platine, 1,3% en poids de germanium et 1% en poids d'indium.

### Exemple 3

Une composition catalytique C supportée sur alumine et contenant du platine, de l'indium et du lithium, a été préparée selon le mode de préparation suivant :
un support alumine est imprégné avec une solution de nitrate de lithium (10 g d'alumine et 20 ml d'eau contenant 0,2 g de LiNO₃. Après évaporation de l'eau à 80 °C puis séchage à 120 °C sous air, le solide est imprégné avec 10 cm³ d'une solution contenant 0,074 g de Pt(NH₃)₄(OH)₂ et 0,136 g de nitrate d'indium In(NO₃)₃. Après évaporation à 80 °C, le solide est calciné sous courant d'air, la température étant augmentée de 25 à 300 °C. Après un palier à 300 °C de 3 heures, le solide est refroidi à 25 °C puis réduit sous hydrogène, la température augmentant jusqu'à 550 °C.

La composition catalytique C ainsi préparée contient, sur alumine, 0,5% en poids de platine, 0,4% en poids d'indium et 0,2% en poids de lithium.

### Exemple 4

La composition D (selon l'art antérieur) est préparée comme décrit dans le document FR 2 115 860, avec en outre 7 mg d'oxyde de niobium Nb₂O₅.

Cette composition D contient donc, sur alumine, 0,5% en poids de platine, 0,4% en poids d'indium, 0,2% en poids de lithium et 5 % en poids de niobium.

### Exemple 5

La composition E selon l'invention est similaire à la composition A selon l'art antérieur, à la différence près qu'elle contient 15 mg d'oxyde de niobium Nb₂O₅.

Cette composition E contient donc, sur silicalite, 0,5 % en poids de platine et 11,6 % en poids de niobium.

### Exemple 6

La composition F selon l'invention est préparée par ajout de 15 mg d'oxyde de niobium Nb₂O₅ à la composition B selon l'art antérieur.

Cette composition F contient donc, sur silicalite, 0,5 % en poids de platine 1,3 % en poids de germanium, 1 % en poids d'indium et 11,6 % en poids de niobium.

### Exemple 7

La composition G selon l'invention est similaire aux compositions C et D, à la différence près qu'elle contient 15 mg d'oxyde de niobium Nb₂O₅.

Cette composition G contient donc, sur alumine, 0,5% en poids de platine, 0,4% en poids d'indium, 0,2% en poids de lithium et 11,6 % en poids de niobium.

### Exemple 8

La composition H selon l'invention est similaire aux compositions C et D, à la différence près qu'elle contient 45 mg d'oxyde de niobium Nb₂O₅.

Cette composition H contient donc, sur alumine, 0,5% en poids de platine, 0,4% en poids d'indium, 0,2% en poids de lithium et 25 % en poids de niobium.

### Essais

Afin d'illustrer les propriétés des compositions catalytiques de l'invention, les compositions E, F, G et H selon l'invention ont été testées et comparées aux compositions A, B, C et D de l'art antérieur.

### Test n°1

Il s'agit de tests de déshydrogénation du propane qui ont été accomplis dans les mêmes conditions en employant la composition catalytique A, conformément à l'art antérieur, puis la composition catalytique E, conformément à l'invention. Les réactions ont été effectuées en présence de 75 mg de la composition catalytique A à une température de l'ordre de 550 °C, à une pression de 10⁵ Pa, sans ajout d'H₂ exogène. La pph est égale à 13 h⁻¹.

Les résultats de conversion du propane sont rassemblés dans le Tableau I en mesurant au cours du temps la conversion en % massique de propane converti et la sélectivité en % massique de propène obtenu.

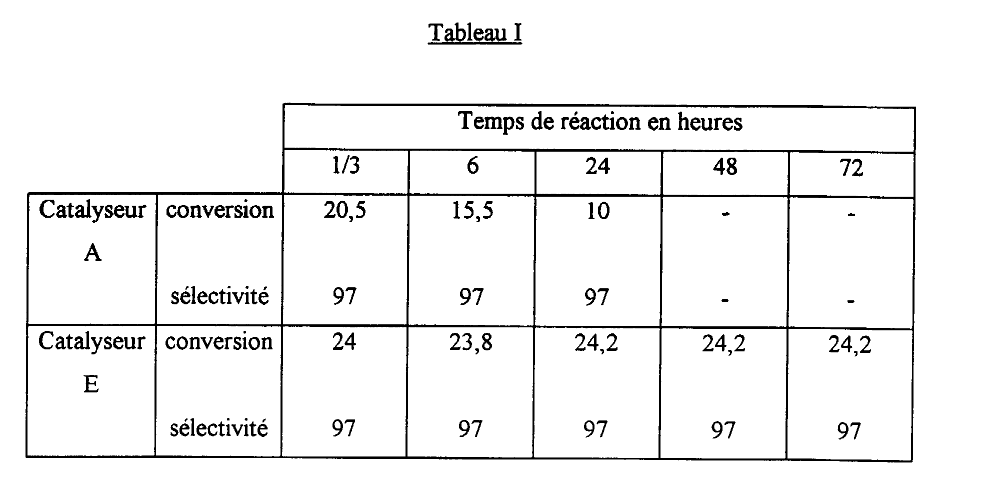

On constate que l'emploi de la composition E contenant du niobium permet une conversion très sensiblement meilleure par rapport à l'emploi de la composition A, en terme de stabilité dans le temps.

### Test n° 2

Il s'agit du même test de déshydrogénation du propane que le test n° 1 mais en utilisant les compositions catalytiques B (art antérieur) et F (selon l'invention).

Les résultats de la conversion du propane sont rassemblés dans le Tableau II en mesurant au cours du temps la conversion en % massique de propane converti et la sélectivité en % massique de propène obtenu.

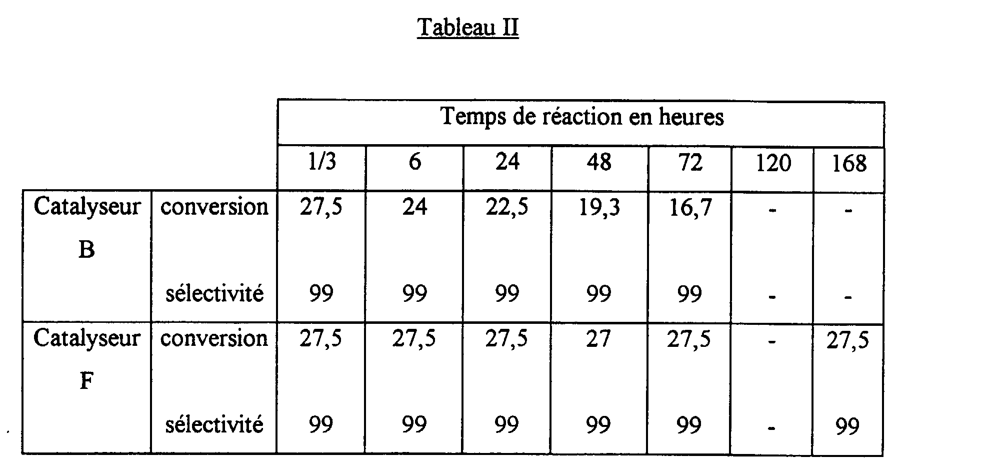

On constate que l'emploi de la composition F contenant du niobium permet une conversion très sensiblement meilleure par rapport à l'emploi de la composition B en terme de stabilité dans le temps.

### Test n°3

Il s'agit du même test de déshydrogénation que le test n°2 mais sur une période plus longue et en fonction de la pression de réaction.

Les résultats de conversion du propane sont rassemblés dans le Tableau III en mesurant au cours du temps la conversion en % massique de propane converti et la sélectivité en % massique de propène obtenu.

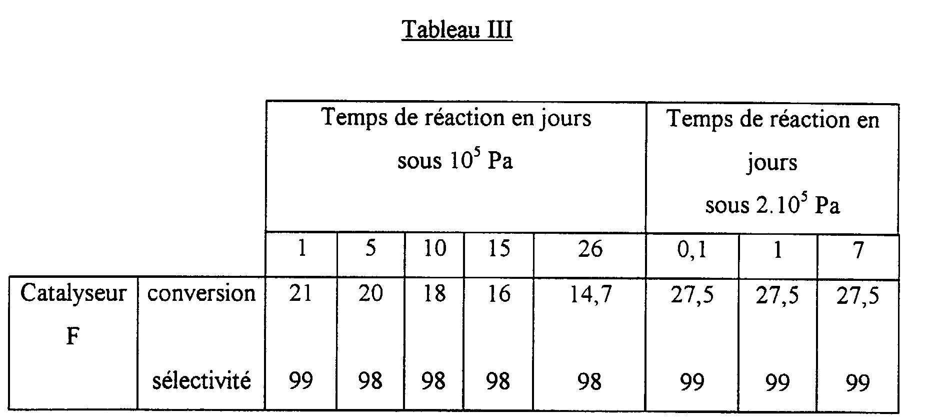

En utilisant la composition catalytique F conformémént à l'invention, on constate que la conversion et la sélectivité demeurent très élevées au-delà de plusieurs semaines et traduisent une activité et une stabilité remarquables et inégalées par rapport aux compositions catalytiques de l'art antérieur.

### Test n°4

Il s'agit du même test de déshydrogénation que le test n°3 mais en comparant les compositions catalytiques C et D (art antérieur) avec les compositions catalytiques G et H (selon l'invention).

Les résultats de conversion du propane sont rassemblés dans le Tableau IV en mesurant au cours du temps la conversion en % massique de propane converti et la sélectivité en % massique de propène obtenu.

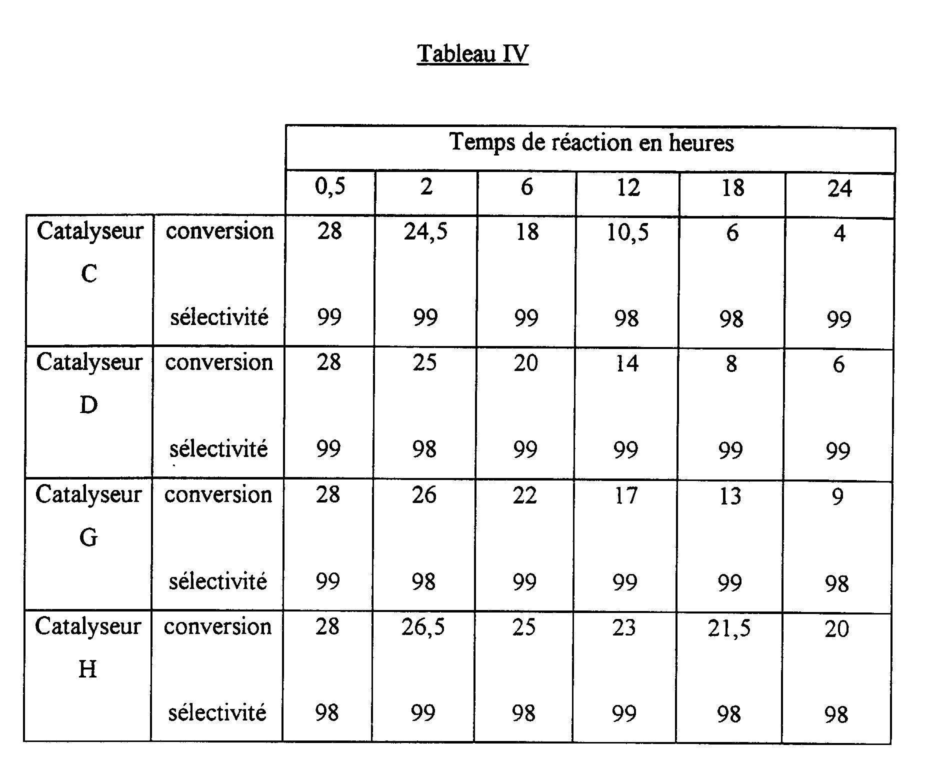

La conversion en présence des compositions catalytiques G et H ayant pour support de l'alumine demeure meilleure que celle des compositions catalytiques C et D de l'art antérieur, tandis que la sélectivité reste excellente.

## Revendications

1. Procédé de traitement d'une charge hydrocarbonée contenant une quantité substantielle d'alcanes, par déshydrogénation d'au moins une fraction desdits alcanes en alcènes et hydrogène, procédé dans lequel on fait passer la charge dans au moins un réacteur en présence d'au moins un catalyseur de déshydrogénation, ledit procédé étant caractérisé en ce que l'on emploie un catalyseur comprenant un support d'oxyde réfractaire inorganique, au moins un métal du groupe du platine ainsi que du niobium sous forme d'oxyde et à une teneur comprise entre 5% et 70% en poids de niobium par rapport au poids dudit catalyseur.

2. Procédé selon la revendication 1, caractérisé en ce que le catalyseur employé contient de 0,01 à 5 % en poids de métaux du groupe du platine.

3. Procédé selon la revendication 2, caractérisé en ce que le métal du groupe du platine est le platine et en ce que le rapport massique Nb/Pt est compris entre 1 et 7000 et de préférence entre 2,5 et 5000.

4. Procédé selon l'une quelconque des revendication précédentes, caractérisé en ce que le support du catalyseur employé comprend au moins un oxyde réfractaire inorganique d'un élément du groupe II, III, ou IV de la classification périodique des éléments, un mélange de ces oxydes ou un composé contenant un ou plusieurs de ces oxydes, les oxydes individuels pouvant être une silice, une alumine, une silice-alumine, une silicalite, un alumino-silicate, une magnésie, une zircone, une silice-oxyde de magnésium ou une alumine-oxyde de bore.

5. Procédé selon l'une quelconque des revendication précédentes, caractérisé en ce qu'il est mis en oeuvre à une température comprise entre 300 et 800°C, à une pression comprise entre 10³ Pa et 10⁶ Pa et à un débit massique de la charge par rapport à la masse de catalyseur (pph) compris entre 1 et 50 h⁻¹.

6. Procédé selon l'une quelconque des revendication précédentes, caractérisé en ce que la température est comprise entre 500°C et 600°C et la pression est de l'ordre de 10⁵ Pa.

7. Procédé selon l'une quelconque des revendication précédentes, caractérisé en ce que lesdits alcanes comprennent du propane et lesdits alcènes comprennent du propène.

8. Procédé selon l'une quelconque des revendication précédentes, caractérisé en ce que l'on utilise un gaz diluant tel que par exemple l'hydrogène, la vapeur d'eau, un gaz inerte, du méthane.

9. Composition catalytique pour la déshydrogénation des alcanes en alcènes et hydrogène, contenant un support de silicalite, au moins un métal du groupe du platine à une teneur comprise entre 0,01 et 5 % en poids, et caractérisée en ce qu'elle contient également du niobium sous forme d'oxyde et à une teneur comprise entre 5% et 70% en poids de niobium par rapport au poids du catalyseur.

10. Composition catalytique selon la revendication 9, caractérisée en ce que le métal du groupe du platine est le platine et en ce que le rapport massique Nb/Pt est compris entre 1 et 7000 et de préférence entre 2,5 et 5000.

11. Composition catalytique selon l'une des revendications 9 et 10, caractérisée en ce qu'elle comprend en outre de 0,01 à 10 % en poids de germanium.

12. Composition catalytique selon la revendication 11, caractérisée en ce qu'elle comprend de 0,01 à 2 % en poids de germanium.

13. Composition catalytique selon l'une des revendications 9 à 12, caractérisée en ce qu'elle comprend en outre de 0,01 à 10 % en poids d'étain.

14. Composition catalytique selon la revendication 13, caractérisée en ce qu'elle comprend de 0,01 à 2 % en poids d'étain.

15. Composition catalytique selon l'une des revendications 9 à 14, caractérisée en ce qu'elle comprend en outre de 0,01 à 10 % en poids d'indium.

16. Composition catalytique selon la revendication 15, caractérisée en ce qu'elle comprend de 0,01 à 2 % en poids d'indium.

17. Procédé de préparation et d'activation d'une composition catalytique de déshydrogénation des alcanes en alcènes et hydrogène, caractérisé en ce qu'il comprend les étapes suivantes:
(1) formation d'un support catalytique contenant éventuellement du germanium, de l'indium et/ou de l'étain, en présence d'un agent structurant;
(2) après cristallisation du mélange homogène à pression autogène et à une température comprise entre 25 et 190°C, filtration et lavage à l'eau du matériau solide, puis séchage;
(3) calcination du matériau séché, sous atmosphère inerte, puis en présence d'oxygène ou d'air, à une température comprise entre 400 et 700°C;
(4) imprégnation du matériau calciné refroidi avec une solution d'au moins une source d'un métal du groupe du platine, sous agitation constante, à une température comprise entre 15 et 95°C ;
(5) évaporation de la phase liquide;
(6) activation du solide récupéré par (i) traitement sous oxygène ou air à une température croissante de 15 à 320°C et (ii) traitement sous hydrogène à une température croissante de 320 à 550°C; et,
(7) refroidissement du catalyseur obtenu;
(8) mélange mécanique du catalyseur obtenu avec de l'oxyde de niobium.

18. Procédé de préparation et d'activation d'une composition catalytique selon la revendication 17, caractérisé en ce que dans l'étape (1), le support catalytique formé contient en outre du niobium.

19. Procédé selon l'une des revendications 17 et 18, caractérisé en ce que la source du métal du groupe du platine est choisie parmi l'acide chloroplatinique PtH₂Cl₆et les sels de platine solubles tels que Pt(NH₃)₄(OH)₂ et Pt(NH₃)₄Cl₂.
